# EUROPEAN PATENT SPECIFICATION

(11) **EP 1 853 217 B1**
(45) Date of publication and mention of the grant of the patent: **01.04.2009**
(21) Application number: 06710920.7
(22) Date of filing: 16.02.2006
(51) Int. Cl.: A61K 8/60, A61Q 5/04

(54) **HAIRCARE USE OF C-GLYCOSIDE DERIVATIVES**
VERWENDUNG VON C-GLYCOSID-DERIVATEN ZUR HAARPFLEGE
SOIN CAPILLAIRE A BASE DE DERIVES C-GLYCOSIDE

(30) Priority: 25.02.2005 FR 0550517; 20.04.2005 US 672990 P
(43) Date of publication of application: 14.11.2007
(73) Proprietor: L'Oréal, 75008 Paris (FR)
(72) Inventor: LEROY, Frédéric, F-92210 Saint Cloud (FR); BARBARAT, Philippe, F-92270 Bois-colombes (FR); DALKO, Maria, F-91190 Gif Sur Yvette (FR)
(74) Representative: Le Coupanec, Pascale A.M.P.
(86) International application number: PCT/IB2006/050507
(87) International publication number: WO 2006/090307

(56) References cited:
- WO-A-02/051803
- WO-A-02/051828
- FR-A- 2 770 776

## Description

The present invention relates to the use of a particular family of C-glycoside derivatives in the field of haircare, especially for increasing the mechanical strength of keratin fibers and more particularly for preventing breaking of the hair.

In the field of haircare, problems of breaking of the hair frequently arise when the hair is subjected to a simple mechanical treatment such as brushing, combing or straightening, this type of treatment possibly being performed in combination with concomitant or consecutive cosmetic treatments, for instance relaxing, shampooing, dyeing or permanent-waving of the hair.

The problems of breaking are moreover particularly exacerbated on certain very curly hair types, where a phenomenon of premature breaking is observed (J. Soc. Cosmet. Chem., 36, January/February 1985, 39-52). The high degree of curliness of this type of hair opposes easy disentangling or straightening and thus requires the application of larger mechanical tensions. For obvious reasons, these tensions significantly affect the mechanical strength of the hair and generally give rise to considerable breakage.

To avoid the problems of breaking of the hair, it has already been proposed to treat the hair, and more particularly the premature breaking of very curly hair, especially of African type, with aqueous solutions of humectants, such as glycerol, glycerol combined with urea, a poly(acrylic acid), dimethyl sulfoxide and a poly(acrylic acid) combined with dimethyl sulfoxide (J. Soc. Cosmet. Chem., 36, January/February 1985, 39-52).

The inventors have found, unexpectedly, that a particular family of C-glycosides is particularly advantageous for preventing this phenomenon of breaking of the hair.

More specifically, they have found that the use of certain C-glycoside derivatives in the field of haircare, especially on natural or treated hair, makes it possible to improve its mechanical strength and thus to significantly prevent its breaking.

Consequently, according to a first aspect, one subject of the present invention is the cosmetic use of at least one compound of general formula (I): 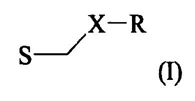 in which:
- X represents a radical chosen from the groups: 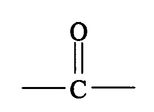 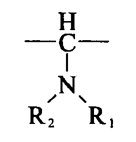 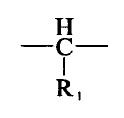 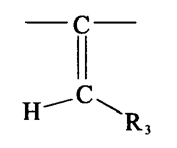 with R₁, R₂ and R₃ representing, independently of each other, a hydrogen atom, an OH group or a radical R with R as defined below,
- R represents:
   - a saturated or unsaturated, linear, branched or cyclic C₁ to C₂₀ alkyl radical,
   - a saturated or unsaturated, linear, branched or cyclic C₁ to C₂₀ polyfluoroalkyl or perfluoroalkyl radical, or
   - a C₅ to C₂₀ aryl radical, especially phenyl, or alkylaryl radical, in particular benzyl,
the hydrocarbon-based chain constituting said radicals possibly, where appropriate, being interrupted with 1, 2, 3 or more hetero atoms chosen from:
- oxygen,
- sulfur,
- nitrogen, and
- silicon,
and possibly being substituted with at least one radical chosen from:
- -OR₄,
- -SR₄,
- -NR₄R₅,
- -COOR₄,
- CONHR₄,
- CN,
- a halogen atom,
- a C₁ to C₆ polyfluoroalkyl or perfluoroalkyl radical,
- a C₃ to C₈ cycloalkyl or heterocycloalkyl radical, and
- an optionally substituted C₅ to C₁₈ aryl radical,
with R₄ and R₅ possibly representing, independently of each other, a hydrogen atom, a hydroxyl radical or a saturated or unsaturated, linear or branched C₁ to C₃₀ and especially C₁ to C₁₂ alkyl, acyl, perfluoroalkyl or polyfluoroalkyl radical,
- S represents a monosaccharide or a polysaccharide comprising upto 20 sugar units and in particular up to 6 sugar units, in pyranose and/or furanose form and of L and/or D series, said monosaccharide or polysaccharide possibly being substituted with a radical (CH₂)-OR₆, with R₆ representing a hydrogen atom or a C₁ to C₆ alkyl radical, with a hydroxyl group and/or with an O-glycoside radical, and containing at least one free hydroxyl function and/or an optionally protected amine function, and
- the bond S-C represents a bond of C-anomeric nature,
or a salt or isomer thereof, for increasing the mechanical strength of keratin fibers.

In particular, a subject of the present invention is the use of a compound of general formula (I) as defined above for the preparation of a cosmetic composition for increasing the mechanical strength of keratin fibers.

For the purposes of the present invention, the term "keratin fiber" denotes the hair, the eyelashes, the eyebrows and the nails, and more particularly human eyelashes and hair.

According to another aspect, a subject of the present invention is the use of a compound of general formula (I) as defined above for the preparation of a cosmetic composition for preventing breaking of the hair.

According to yet another aspect, a subject of the present invention is the use of a compound of general formula (I) as defined above for the preparation of a cosmetic composition for preventing breaking of the hair.

C-Glycoside derivatives have already been proposed for inducing chondroitin biosynthesis (US 4 446 313 and US 4 454 123).

More recently, document WO 02/051828 has shown that such compounds are particularly effective for stimulating the synthesis of glycosaminoglycans containing a D-glucosamine and/or N-acetyl-D-glucosamine residue.

On the other hand, to the inventors' knowledge, no C-glycoside derivative has been proposed for the purposes of increasing the mechanical strength of keratin fibers, especially human keratin fibers, and in particular for reducing the phenomenon of breaking of the hair.

Advantageously, the monosaccharide represented by S in formula (I) is chosen from D-glucose, D-galactose, D-mannose, D-xylose, D-lyxose, L-fucose, D-fucose, L-arabinose, D-maltose, L-rhamnose, D-glucuronic acid, D-galacturonic acid, D-iduronic acid, N-acetyl-D-glucosamine and N-acetyl-D-galactosamine.

As regards the polysaccharides, they may contain upto 6 sugar units and are especially chosen from D-maltose, D-lactose, D-cellobiose, D-maltotriose, a disaccharide combining a uronic acid chosen from D-iduronic acid and D-glucuronic acid with a hexosamine chosen from D-galactosamine, D-glucosamine, N-acetyl-D-galactosamine and N-acetyl-D-glucosamine, an oligosaccharide containing at least one xylose advantageously chosen from xylobiose, methyl-β-xylobioside, xylotriose, xylotetrose, xylopentose and xylohexose, and preferentially xylobiose, which is composed of two xylose molecules linked via a 1-4 bond.

More particularly, S represents a monosaccharide chosen from D-glucose, D-xylose, D-fucose, D-galactose and D-maltose, and especially D-xylose.

According to one even more preferential form of the invention, the compound of formula (I) is a C-glycoside derivative corresponding to formula (II): 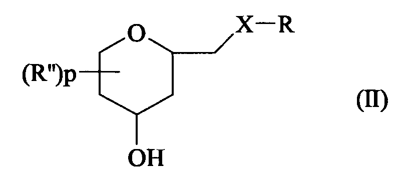 in which:
- p represents an integer chosen from 2 and 3,
- R and X are as defined above, and
- R" represents:
   - a radical (CH₂)-OR₆, with R₆ representing a hydrogen atom or a C₁ to C₆ and in particular C₁ to C₄ alkyl radical,
   - a hydroxyl group, and/or
   - an O-glycoside radical.

The C-anomeric bond in formulae (I) and (II) may be α or β.

Preferably, X represents in formulae (I) and (II) a function: 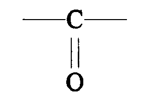 or a unit:

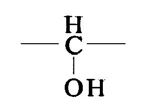

According to another particular mode, R represents therein a saturated, linear or branched C₁ to C₆ alkyl radical and especially a methyl radical.

The invention also covers the optical and/or geometrical isomers of the compounds of formulae (I) and (II), alone or as a mixture in all proportions, and also the physiologically acceptable salts of these compounds.

The C-glycoside derivatives in accordance with the invention may be used alone or has a mixture and in any proportion.

For the purposes of the present invention, the term "mixture" covers mixtures of the various isomeric forms of the same compound and also mixtures of different compounds of general formula I and/or of the respective isomeric forms thereof.

The C-glycoside derivatives may be of natural or synthetic origin, totally or partially purified from any preparation containing them.

The term "natural origin" means a derivative extracted from a natural material, for instance a plant. The term "synthetic origin" means a derivative prepared via chemical or synthesis or biotechnology.

The expression "totally or partially purified" means herein that, during its synthesis or in comparison with its natural state (fresh or dried plant or cells), the compounds of formulae (I) and (II) according to the invention have been concentrated and/or have been freed, respectively, of at least some of the side reaction products derived from their synthesis or of at least some of the other constituents of the natural material in which they are present.

As nonlimiting illustrations of compounds that are suitable for the invention, mention may be made especially of the following compounds:
- C-β-D-xylopyranoside-n-propan-2-one,
- C-α- D-xylopyranoside-n-propan-2-one,
- C-β-D-xylopyranoside-2-hydroxypropane,
- C-α- D-xylopyranoside-2-hydroxypropane,
- 1-(C-β-D-fucopyranoside)propan-2-one,
- 1-(C-α-D-fucopyranoside)propan-2-one,
- 1-(C-β-L-fucopyranoside)propan-2-one,
- 1-(C-α-L-fucopyranoside)propan-2-one,
- 1-(C-β-D-fucopyranoside)-2-hydroxypropane,
- 1-(C-α-D-fucopyranoside)-2-hydroxypropane,
- 1-(C-β-L-fucopyranoside)-2-hydroxypropane,
- 1-(C-α-L-fucopyranoside) -2-hydroxypropane,
- 1-(C-β-D-glucopxanosyl)-2-hydroxypropane,
- 1-(C-α-D-glucopyranosyl)-2-hydroxypropane,
- 1-(C-β-D-galactopyranosyl)-2-hydroxypropane,
- 1-(C-α-D-galactopyranosyl)-2-hydroxypropane
- 1-(C-β-D-fucofuranosyl)propan-2-one,
- 1-(C-α-D-fucofuranosyl)propan-2-one
- 1-(C-β-L-fucofuranosyl)propan-2-one,
- 1-(C-α-L-fucofuranosyl)propan-2-one,
- C-β-D-maltopyranoside-n-propan-2-one,
- C-α-D-maltopyranoside-n-propan-2-one
- C-β-D-maltopyranoside-2-hydroxypropane,
- C-α-D-maltopyranoside-2-hydroxypropane, isomers thereof and mixtures thereof.

The compounds of general formula (I) are generally formulated in the form of a cosmetic composition intended to be used for increasing the mechanical strength of keratin fibers and/or for preventing breaking of the hair.

The compositions generally contain a physiologically acceptable medium in which is formulated one or more compounds according to the invention in an amount effective for increasing the mechanical strength of keratin fibers and/or for preventing the phenomenon of breaking of the hair.

For example, this amount may range from 0.0001% to 30% by weight and preferably from 0.0001% to 5% by weight relative to the total weight of the composition.

The term " physiologically acceptable medium" comprises a medium that is compatible with the skin, mucous membranes, the nails, the scalp and/or the hair.

The composition according to the invention may especially be in the form of an aqueous solution or a dispersion of the lotion or serum type, emulsions of liquid or semiliquid consistency of the milk type, obtained by dispersing a fatty phase in an aqueous phase (O/W) or conversely (W/O), or suspensions or emulsions of soft consistency of the aqueous or anhydrous cream or gel type, or alternatively microcapsules, microparticles or vesicular dispersions of ionic and/or nonionic type. These compositions are prepared according to the usual methods.

This composition may be more or less fluid and may have the appearance of a white or colored cream, a pomade, a milk, a lotion, a serum, a paste or a mousse. It may optionally be applied in aerosol form. It may also be in solid form, for example in stick form It may be used as a care product, as a cleansing product or as a shampoo or hair conditioner.

When the composition that may be used according to the invention is an emulsion, the proportion of the fatty phase may range from 5% to 80% by weight and preferably from 5% to 50% by weight relative to the total weight of the composition. The oils, waxes, emulsifiers and coemulsifiers used in the composition in emulsion form are chosen from those conventionally used in cosmetics. The emulsifier and the coemulsifier are present in the composition in a proportion ranging from 0.3% to 30% by weight and preferably from 0.5% to 20% by weight relative to the total weight of the composition. The emulsion may also contain lipid vesicles.

As oils or waxes that may be used in the invention, mention may be made of mineral oils (liquid petroleum jelly), plant oils (liquid fraction of shea butter, or sunflower oil), animal oils (perhydrosqualene), synthetic oils (purcellin oil), silicone oils or waxes (cyclomethicone) and fluoro oils (perfluoropolyethers), beeswax, carnauba wax or paraffin wax. Fatty alcohols and fatty acids (stearic acid) may be added to these oils.

As emulsifiers that may be used in the invention, examples that may be mentioned include glyceryl monostearate, polysorbate 60 and polyethylene glycol stearates (20 EO, 40 EO and 100 EO).

In a known manner, the composition of the invention may also contain adjuvants that are common in cosmetics and haircare, such as hydrophilic or lipophilic gelling agents, hydrophilic or lipophilic active agents, preserving agents, antioxidants, solvents, fragrances, fillers, screening agents, pigments, chelating agents and dyestuffs. The amounts of these various adjuvants are those conventionally used in the fields under consideration, for example from 0.01% to 20% of the total weight of the composition. Depending on their nature, these adjuvants may be introduced into the fatty phase, into the aqueous phase, into lipid vesicles and/or into nanoparticles. They are chosen so as not to harm the properties of the compounds according to the invention.

As hydrophilic gelling agents that may be used in the invention, mention may be made of carboxyvinyl polymers (carbomer), acrylic copolymers such as acrylate/alkylacrylate copolymers, polyacrylamides, polysaccharides such as hydroxypropylcellulose, natural gums and clays, and, as lipophilic gelling agents, mention may be made of modified clays, for instance bentones, metal salts of fatty acids, for instance aluminum stearates, and hydrophobic silica.

A subject of the invention is thus also a process for the cosmetic treatment of hair fibers, comprising the application to the hair fibers of a composition in accordance with the invention.

This process is in particular intended for preventing the phenomenon of breaking of the hair.

A subject of the invention is thus also a process for the cosmetic treatment of the hair, comprising the application to the hair of a composition as defined above to wet or dry hair, followed by optional rinsing.

Other characteristics and advantages of the invention will emerge more clearly from the examples that follow, which are given as nonlimiting illustrations. In the text hereinbelow, the proportions are given as weight percentages, unless otherwise indicated.

### Example 1:

C-β-D-Xylopyranoside-n-propan-2-one (compound 2) and C-β-D-xylopyranoside-2-hydroxypropane (compound 1) were prepared, respectively, according to the synthetic protocols described in examples 1 and 4 of patent application WO 02/051828.

C-β-D-Lactopyranoside-n-propan-2-one (compound 3) and C-β-D-glucopyranoside-n-propan-2-one (compound 4) were prepared by reproducing the protocol proposed for compound 2, replacing the xylose therein with lactose and glucose, respectively.

### Example 2:

The hair used for the following tests was regulated beforehand in a glove box at 25°C and 45% relative humidity for about 24 hours. This was natural curly African hair.

The four abovementioned compounds were respectively formulated in the form of a composition comprising 5% by weight of the compound under consideration and 95% by weight of water relative to the total weight of the composition.

Four batches of natural African hair were treated, respectively, with each of the four compositions according to the following protocol: the hair is immersed for three days at 40°C in the composition under consideration, without final rinsing, and its degree of premature breaking is then assessed by comparison with that of a batch of untreated natural control hair.

The degree of premature breaking of the hair is characterized by the following parameter R:
- R = 1 - (number of hairs showing breakage under tension in the hardening zone/N),
- N being the total number of hairs tested.

The corresponding mechanical tests are performed using a Zwick^{®} tensile testing machine on hairs 30 mm long. The hairs were pulled at a speed of 10 mm/minute.

The results are collated in the following table:

| **Hair treatment product** | **Degree of premature breaking** |
|---|---|
| None (control) | 0.27 |
| Compound 1 | 0.17 |
| Compound 2 | 0.20 |
| Compound 3 | 0.13 |
| Compound 4 | 0.23 |

The compositions according to the invention allow the degree of premature breaking of the hair to be reduced.

### Example 3

| Hair lotion: | | weight% |
|---|---|---|
| - Compound 1 | | 1 |
| - Propylene glycol | | 30 |
| - Ethyl alcohol | | 40 |
| - Water | qs | 100 |

## Claims

1. A cosmetic use of at least one compound of general formula (I): 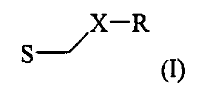 in which:
- X represents a radical chosen from the groups: 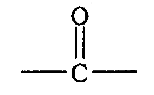 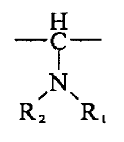 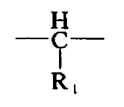 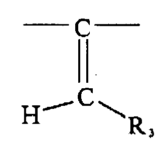 with R₁, R₂ and R₃ representing, independently of each other, a hydrogen atom, an OH group or a radical R with R as defined below,
- R represents:
- a saturated or unsaturated, linear, branched or cyclic C₁ to C₂₀ alkyl radical,
- a saturated or unsaturated, linear, branched or cyclic C₁ to C₂₀ polyfluoroalkyl or perfluoroalkyl radical, or
- a C₅ to C₂₀ aryl radical, especially phenyl, or alkylaryl radical, in particular benzyl,
the hydrocarbon-based chain constituting said radicals possibly, where appropriate, being interrupted with 1, 2, 3 or more hetero atoms chosen from:
- oxygen,
- sulfur,
- nitrogen, and
- silicon,
and possibly being substituted with at least one radical chosen from:
- -OR₄,
- -SR₄,
- -NR₄R₅,
- -COOR₄,
- -CONHR₄,
- CN,
- a halogen atom,
- a C₁ to C₆ polyfluoroalkyl or perfluoroalkyl radical,
- a C₃ to C₈ cycloalkyl or heterocycloalkyl radical, and
- a C₅ to C₁₈ aryl radical,
with R₄ and R₅ possibly representing, independently of each other, a hydrogen atom, a hydroxyl radical or a saturated or unsaturated, linear or branched C₁ to C₃₀ and especially C₁ to C₁₂ alkyl, acyl, perfluoroalkyl or polyfluoroalkyl radical,
- S represents a monosaccharide or a polysaccharide comprising upto 20 sugar units, in pyranose and/or furanose form and of L and/or D series, said monosaccharide or polysaccharide possibly being substituted with a radical (CH₂)-OR₆, with R₆ representing a hydrogen atom or a C₁ to C₆ alkyl radical, with a hydroxyl group and/or with an O-glycoside radical, and containing at least one free hydroxyl function and/or an optionally protected amine function, and
- the bond S-C represents a bond of C-anomeric nature,
or a salt or isomer thereof, for increasing the mechanical strength of keratin fibers.

2. The use as claimed in claim 1, **characterized in that** the keratin fibers are human hair and/or eyelashes.

3. The cosmetic use of at least one compound as defined in claim 1 for preventing breaking of the hair.

4. The use as claimed in any one of claims 1 to 3, **characterized in that** S represents a monosaccharide chosen from D-glucose, D-galactose, D-mannose, D-xylose, D-lyxose, L-fucose, D-fucose, L-arabinose, D-maltose, L-rhamnose, D-glucuronic acid, D-galacturonic acid, D-iduronic acid, N-acetyl-D-glucosamine and N-acetyl-D-galactosamine.

5. The use as claimed in claim 4, **characterized in that** S represents a monosaccharide chosen from D-glucose, D-xylose, D-fucose, D-galactose and D-maltose, and especially D-xylose.

6. The use as claimed in any one of claims 1 to 5, **characterized in that** the compound of general formula (I) is a C-glycoside derivative of general formula (II): 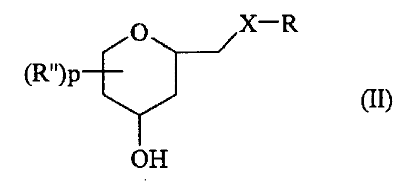 in which:
- p represents an integer chosen from 2 and 3,
- R and X are as defined in claim 1, and
- R" represents:
- a radical (CH₂)-OR₆, with R₆ representing a hydrogen atom or a C₁ to C₆ and in particular C₁ to C₄ alkyl radical,
- a hydroxyl group, and/or
- an O-glycoside radical.

7. The use as claimed in any one of claims 1 to 6, **characterized in that** "X" represents a carbonyl function or a -CHOH- group.

8. The use as claimed in any one of claims 1 to 7, **characterized in that** R represents a saturated, linear or branched C₁ to C₆ alkyl radical and in particular a methyl radical.

9. The use as claimed in any one of the preceding claims, **characterized in that** said compound of general formula I is chosen from:
- C-β-D-xylopyranoside-n-propan-2-one,
- C-α- D-xylopyranoside-n-propan-2-one,
- C-β-D-xylopyranoside-2-hydroxypropane,
- C-α- D-xylopyranoside-2-hydroxypropane,
- 1-(C-β-D-fucopyranoside)propan-2-one,
- 1-(C-α-D-fucopyranoside)propan-2-one,
- 1-(C-β-L-fucopyranosidc)propan-2-one,
- 1-(C-α-L-fucopyranoside)propan-2-one,
- 1-(C-β-D-fucopyranoside)-2-hydroxypropane,
- 1-(C-α-D-fucopyranoside)-2-hydroxypropane,
- 1-(C-β-L-fucopyranoside)-2-hydroxypropane,
- 1-(C-α-L-fucopyranoside) -2-hydroxypropane,
- 1-(C-β-D-glucopyranosyl)-2-hydroxypropane,
- 1-(C-α-D-glucopyranosyl)-2-hydroxypropane,
- 1-(C-β-D-galactopyranosyl)-2-hydroxypropane,
- 1-(C-α-D-galactopyranosyl)-2-hydroxypropane
- 1-(C-β-D-fucofuranosyl)propan-2-one,
- 1-(C-α-D-fucofuranosyl)propan-2-one
- 1-(C-β-L-fucofuranosyl)propan-2-one,
- 1-(C-α-L-fucofuranosyl)propan-2-one,
- C-β-D-maltopyranoside-n-propan-2-one,
- C-α-D-maltopyranoside-n-propan-2-one
- C-β-D-maltopyranoside-2-hydroxypropane,
- C-α-D-maltopyranoside-2-hydroxypropane,
- isomers thereof and mixtures thereof.

## Patentansprüche

1. Kosmetische Verwendung von mindestens einer Verbindung der allgemeinen Formel (I): 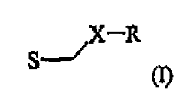 wobei:
- X einen Rest darstellt, welcher ausgewählt ist aus den Resten: 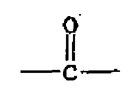 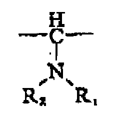 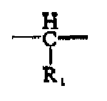 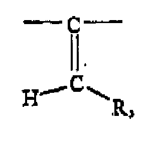 wobei R₁, R₂ und R₃ unabhangig voneinander ein Wasserstoffatom, eine Gruppe OH oder einen Rest R darstellen, wobei R wie nachstehend definiert ist,
- R darstellt:
- einen gesättigten oder ungesättigten, linearen, verzweigten oder cyclischen C₁- bis C₂₀-Alkylrest,
- einen gesättigten oder ungesättigten, linearen, verzweigten oder cyclischen C₁- bis C₂₀-Polyfluoralkyl- oder -Perfluoralkylrest, oder
- einen C₅- bis C₂₀-Arylrest, insbesondere Phenyl, oder Alkylarylrest, insbesondere Benzyl,
wobei die auf Kohlenwasserstoff basierende Kette, welche die Reste aufbaut, möglicherweise, wo passend, unterbrochen ist mit 1, 2, 3 oder mehr Heteroatome, ausgewählt aus:
- Sauerstoff,
- Schwefel,
- Stickstoff und
- Silicium,
und möglicherweise substituiert ist mit mindestens einem Rest, ausgewählt aus:
- -OR₄,
- -SR₄,
- -NR₄R₅,
- -COOR₄,
- -CONHR₄,
- CN,
- einem Halogenatom,
- einem, C₁- bis C₆-Polyfluoralkyl- oder -Perfluoxalkylrest,
- einem C₃- bis C₈-Cycloalkyl- oder -Heterocycloalkylrest, und
- einem C₅- bis C₁₈-Arylrest,
wobei R₄ und R₅ möglicherweise unabhängig voneinander ein Wasserstoffatom, eine Hydroxylgruppe oder einen gesättigten oder ungesättigten, linearen oder verzweigten C₁- bis C₃₀- und insbesondere C₁- bis C₁₂-Alkyl-, -Acyl-, -Perfluoralkyl- oder -Polyfluoralkylrest darstellen,
- S ein Monosaccharid oder ein Polysaccharid, umfassend bis zu 20 Zuckereinheiten, in Pyranose- und/oder Furanoseform und von L- und/oder D-Reiben darstellt, wobei das Monosaccharid oder Polysaccharid möglicherweise mit einem Rest (CH₂)-OR₆ substituiert sind, wobei R₆ ein Wasserstoffatom oder einen C₁- bis C₆-Alkylrest darstellt, mit einer Hydroxylgruppe und/oder mit einem O-Glycosidrest, und mindestens eine freie Hydroxylfunktion und/oder eine gegebenenfalls geschützte Aminfunktion enthält, und
- die Bindung S-C eine Bindung von C-anomerer Natur darstellt,
oder ein Salz oder Isomer davon zur Erhöhung der mechanischen Festigkeit von Keratinfasern.

2. Verwendung wie in Anspruch 1 beansprucht, **dadurch gekennzeichnet, dass** die Keratinfasern menschliches Haar und/oder Augenwimpern sind.

3. Kosmetische Verwendung von mindestens einer Verbindung wie in Anspruch 1 definiert zur Vorbeugung von Brechen des Haares.

4. Verwendung wie in einem der Ansprüche 1 bis 3 beansprucht, **dadurch gekennzeichnet, dass** S ein Monosaccharid, ausgewählt aus D-Glucose, D-Galactose, D-Mannose, D-Xylose, D-Lyxose, L-Fucose, D-Fucose, L-Arabinose, D-Maltose, L-Rhamnose, D-Glucuronsäure, D-Galacturonsäure, D-Iduronsäure, N-Acetyl-D-glucosamin und N-Acetyl-D-galactosamin, darstellt.

5. Verwendung wie in Anspruch 4 beansprucht, **dadurch gekennzeichnet, dass** S ein Monosaccharid, ausgewählt aus D-Glucose, D-Xylose, D-Fucose, D-Galactose und D-Maltose, und insbesondere D-Xylose, darstellt.

6. Verwendung wie in einem der Anspräche 1 bis 5 beansprucht, **dadurch gekennzeichnet, dass** die Verbindung der allgemeinen Formel (I) ein C-Glycosid-Derivat der allgemeinen Formel (II) ist; 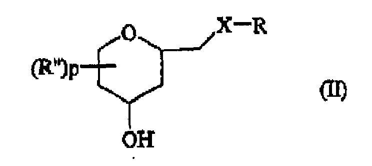 wobei:
- p eine ganze Zahl, ausgewählt aus 2 und 3, darstellt,
- R und X wie in Anspruch 1 definiert sind, und
- R" darstellt:
- einen Rest (CH₂)-OR₆, wobei R₆ ein Wasserstoffatom oder einen C₁- bis C₆- und insbesondere C₁- bis C₄-Alkylrest darstellt,
- eine Hydroxylgruppe, und/oder
- einen 0-Glycosidrest.

7. Verwendung wie in einem der Ansprüche 1 bis 6 beansprucht, **dadurch gekennzeichnet, dass** "X" eine Carbonylfunktion oder eine Gruppe -CHOH- darstellt.

8. Verwendung wie in einem der Ansprüche 1 bis 7 beansprucht, **dadurch gekennzeichnet, dass** R einen gesättigten, linearen oder verzweigten C₁- bis C₆-Alkylrest und insbesondere eine Methylgruppe darstellt.

9. Verwendung wie in einem der vorhergehenden Ansprüche beansprucht, **dadurch gekennzeichnet, dass** die Verbindung der allgemeinen Formel I ausgewählt ist aus:
- C-β-D-Xylopyranosid-n-propan-2-on,
- C-α-D-Xylopyranosid-n-propan-2-on,
- C-β-D-Xylopyzanasid-2-hydroxypropan,
- C-α-D-Xylopyranosid-2-hydroxypropan,
- 1-(C-β-D-Fucopyranosid)propan-2-on,
- 1-(C-α-D-Fucopyranosid)propan-2-on,
- 1-(C-β-L-Fucopyxanosid)propan-2-on,
- 1-(C-α-L-Fucopyranosid)propan-2-on,
- 1-(C-β-D-Fucopyranosid)-2-hydroxypropan,
- 1-(C-α-D-Fucopyranosid)-2-hydroxypropan,
- 1-(C-β-L-Fucopyranosid)-2-hydroxypropan,
- 1-(C-α-L-Fucopyranosid)-2-hydxoxypropan,
- 1-(C-β-D-Glucopyranosyl)-2-hydroxypropan,
- 1-(C-α-D-Glucopyranosyl)-2-hydroxypropan,
- 1-(C-β-D-Galactopyranosyl)-2-hydroxypropan,
- 1-(C-α-D-Galactopyranosyl)-2-hydroxypropan,
- 1-(C-β-D-Fucofuranosyl)propan-2-on,
- 1-(C-α-D-Fucofuranosyl)propan-2-on,
- 1-(C-β-L-Fucofuranosyl)propan-2-on,
- 1-(C-α-L-Fucofuranosyl)propan-2-on,
- C-β-D-Maltopyranosid-n-propan-2-on,
- C-α-D-Maltopyranosid-n-propan-2-on,
- C-β-D-Maltopyranosid-2-hydroxypropan,
- C-α-D-Maltopyranosid-2-hydroxypropan,
- Isomeren davon und Gemischen davon.

## Revendications

1. Utilisation cosmétique d'au moins un composé de formule générale (I) : 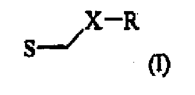 dans laquelle :
- X représente un radical choisi parmi les groupes : 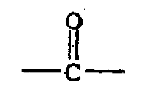 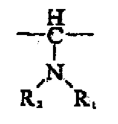 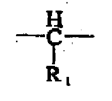 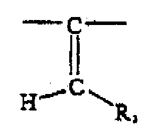 R₁, R₂ et R₃ représentant, indépendamment les uns des autres, un atome d'hydrogène, un groupe OH ou un radical R, R étant tel que défini ci-après,
- R représente :
- un radical alkyle en C₁ à C₂₀ saturé ou insaturé, linéaire, ramifié ou cyclique,
- un radical polyfluoroalkyle ou perfluoroalkyle en C₁ à C₂₀ saturé ou insaturé, linéaire, ramifié ou cyclique, ou
- un radical aryle en C₅ à C₂₀, spécialement phényle, ou un radical alkylaryle, en particulier benzyle,
la chaîne à base d'hydrocarbure constituant lesdits radicaux étant éventuellement, lorsque cela convient, interrompue par 1, 2, 3 hétéroatomes ou plus choisis parmi :
- l'oxygène,
- le soufre,
- l'azote, et
- le silicium,
et étant éventuellement substituée par au moins un radical choisi parmi :
- -OR₄,
- -SR₄,
- -NR₄R₅,
- -COOR₄,
- -CONHR₄,
- CN,
- un atome d'halogène,
- un radical polyfluoroalkyle ou perfluoroalkyle en C₁ à C₆,
- un radical cycloalkyle ou hétérocycloalkyle en C₃ à C₈ , et
- un radical aryle en C₅ à C₁₈,
R₄ et R₅ représentant éventuellement, indépendamment l'un de l'autre, un atome d'hydrogène, un radical hydroxyle ou un radical alkyle, acyle, perfluoroalkyle ou polyfluoroalkyle en C₁ à C₃₀, et spécialement en C₁ à C₁₂, saturé ou insaturé, linéaire ou ramifié,
- S représente un monosaccharide ou un polysaccharide comprenant jusqu'à 20 motifs sucres, sous forme pyranose et/ou furanose et des séries L et/ou D, ledit monosaccharide ou polysaccharide étant éventuellement substitué par un radical (CH₂) -OR₆, R₆ représentant un atome d'hydrogène ou un radical alkyle en C₁ à C₆, par un groupe hydroxyle et/ou par un radical O-glycoside, et contenant au moins une fonction hydroxyle libre et/ou une fonction amine facultativement protégée, et
- la liaison S-C représente une liaison de nature C-anomère,
ou d'un sel ou isomère de celui-ci, pour augmenter la résistance mécanique des fibres de kératine.

2. Utilisation selon la revendication 1, **caractérisée en ce que** les fibres de kératine sont des cheveux et/ou des cils humains.

3. Utilisation cosmétique d'au moins un composé tel que défini dans la revendication 1 pour prévenir une cassure des cheveux.

4. Utilisation selon l'une quelconque des revendications 1 à 3, **caractérisée en ce que** S représente un monosaccharide choisi parmi le D-glucose, le D-galactose, le D-mannose, le D-xylose, le D-lyxose, le L-fucose, le D-fucose, le L-arabinose, le D-maltose, le L-rhamnose, l'acide D-glucuronique, l'acide D-galacturonique, l'acide D-iduronique, la N-acétyl-D-glucosamine et la N-acétyl-D-galactosamine.

5. Utilisation selon la revendication 4, **caractérisée en ce que** S représente un monosaccharide choisi parmi le D-glucose, le D-xylose, le D-fucose, le D-galactose et le D-maltose, et spécialement le D-xylose.

6. Utilisation selon l'une quelconque des revendications 1 à 5, **caractérisée en ce que** le composé de formule générale (I) est un dérivé de C-glycoside de formule générale (II) : 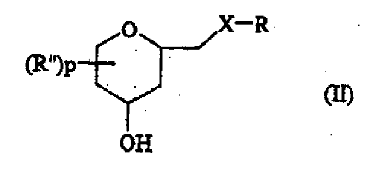 dans laquelle :
- p représente un entier choisi parmi 2 et 3,
- R et X sont tels que définis dans la revendication 1, et
- R" représente :
- un radical (CH₂)-OR₆, R₆ représentant un atome d'hydrogène ou un radical alkyle en C₁ à C₆, et en particulier en C₁ à C₄,
- un groupe hydroxyle, et/ou
- un radical O-glycoside.

7. Utilisation selon l'une quelconque des revendications 1 à 6, **caractérisée en ce que** "X" représente une fonction carbonyle ou un groupe -CHOH-.

8. Utilisation selon l'une quelconque des revendications 1 à 7, **caractérisée en ce que** R représente un radical alkyle en C₁ à C₆ saturé, linéaire ou ramifié et en particulier un radical méthyle.

9. Utilisation selon l'une quelconque des revendications précédentes, **caractérisée en ce que** ledit composé de formule générale I est choisi parmi :
- C-β-D-xylopyranoside-n-propan-2-one,
- C-α-D-xylopyranoside-n-propan-2-one,
- C-β-D-xylopyranoside-2-hydroxypropane,
- C-α- D-xylopyranoside-2-hydroxypropane,
- 1-(C-β-D-fuccopyranoside)propan-2-one,
- 1-(C-α-D-fucopyranoside)propan-2-one,
- 1-(C-β-L-fucopyranoside)propan-2-one,
- 1-(C-α-L-fucopyranoside)propan-2-one,
- 1-(C-β-D-fucopyranoside)-2-hydroxypropane,
- 1-(C-α-D-fucopyranoside)-2-hydroxypropane,
- 1-(C-β-L-fucopyranoside)-2-hydroxypropane,
- 1-(C-α-L-fucopyranoside)-2-hydroxypropane,
- 1-(C-β-D-glucopyranosyl)-2-hydroxypropane,
- 1-(C-α-D-glucopyranosyl)-2-hydroxypropane,
- 1-(C-β-D-galactopyranosyl)-2-hydroxypropane,
- 1-(C-α-D-galactopyranosyl)-2-hydroxypropane
- 1-(C-β-D-fucofuranosyl)propan-2-one,
- 1-(C-α-D-fucofuranosyl)propan-2-one,
- 1-(C-β-L-fucofuranosyl)propan-2-one,
- 1-(C-α-L-fucofuranosyl)propan-2-one,
- C-β-D-maltopyranoside-n-propan-2-one,
- C-α-D-maltopyranoside-n-propan-2-one
- C-β-D-maltopyranoside-2-hydroxypropane,
- C-α-D-maltopyranoside-2-hydroxypropane,
- les isomères de ceux-ci et les mélanges de ceux-ci.
